# EUROPEAN PATENT SPECIFICATION

(11) **EP 2 689 777 B1**
(45) Date of publication and mention of the grant of the patent: **28.03.2018**
(21) Application number: 11861553.3
(22) Date of filing: 22.03.2011
(51) Int. Cl.: A61K 31/352, C07D 311/62, A61P 1/16, A61P 31/12

(54) **PHARMACEUTICAL COMPOSITION FOR INDUCING HEPATIC REGENERATION**
PHARMAZEUTISCHE ZUSAMMENSETZUNG ZUR STIMULATION VON LEBERREGENERATION
COMPOSITION PHARMACEUTIQUE SERVANT À INDUIRE LA REGENERATION HÉPATIQUE

(43) Date of publication of application: 29.01.2014
(73) Proprietor: Industrial Technology Research Institute, Chutung, Hsinchu 31040 (TW)
(72) Inventor: CHANG, Shau-Feng, Hsinchu City Taiwan 30069 (TW); MA, Chun-Hsien, Hsinchu City Taiwan 300 (TW); YANG, Kuo-Yi, Hsinchu City Taiwan 300 (TW); LIN, Shyh-Horng, Kaohsiung City Taiwan 811 (TW); LIN, Chien-Tung, Hsinchu City Taiwan 300 (TW); HUANG, Kai-Wen, Taipei City Taiwan 112 (TW)
(74) Representative: Fuchs Patentanwälte Partnerschaft mbB
(86) International application number: PCT/CN2011/072045
(87) International publication number: WO 2012/126178

(56) References cited:
- CN-A- 1 443 533
- CN-A- 1 933 818
- CN-A- 101 507 730
- CN-A- 101 822 372
- JP-A- 2009 242 374
- US-A1- 2002 151 582
- US-A1- 2005 171 029
- US-A1- 2007 054 868
- WEIGUANG YI ET AL: "Effects of phenolic compounds in blueberries and muscadine grapes on HepG2 cell viability and apoptosis", FOOD RESEARCH INTERNATIONAL., vol. 39, no. 5, 1 June 2006 (2006-06-01), pages 628-638, XP55133684, GB ISSN: 0963-9969, DOI: 10.1016/j.foodres.2006.01.001
- S. J. LEE ET AL: "Phenolic Phytochemicals Derived from Red Pine (Pinus densiflora) Inhibit the Invasion and Migration of SK-Hep-1 Human Hepatocellular Carcinoma Cells", ANNALS OF THE NEW YORK ACADEMY OF SCIENCES, vol. 1095, no. 1, 1 January 2007 (2007-01-01), pages 536-544, XP55133686, ISSN: 0077-8923, DOI: 10.1196/annals.1397.058
- BOMSER J ET AL: "IN VITRO ANTICANCER ACTIVITY OF FRUIT EXTRACTS FROM VACCINIUM SPECIES", PLANTA MEDICA, THIEME VERLAG, DE, vol. 62, no. 3, 1 January 1996 (1996-01-01), pages 212-216, XP008021544, ISSN: 0032-0943

## Description

### TECHNICAL FIELD

The disclosure relates to a pharmaceutical composition, and more particularly, to a pharmaceutical composition for promoting damaged liver regeneration.

### BACKGROUND

Hepatocellular carcinoma (HCC, liver cancer) ranked the fifth among the causes of death of cancer in the world in males, the eighth in females. HCC is nearly indetectable in early stage. Therefore, delay of the best timing of treatment happens all the time. Clinically, hepatectomy surgery or liver transplantation is the best methods for HCC treatment. However, since most HCC patients have been diagnosed in late stage, therefore, only 15% of patients can be treated by hepatectomy surgery, however, its recovery rate is lower than 5%. Additionally, other treatment methods including transcatheter arterial chemoembolization (TACE), radio-frequency ablation (RFA) and radiotherapy can be used, however, its relapse rate is over 80%. When patients with clinical symptoms are diagnosed with HCC, the average survival time thereof is only round 6 months, in light of this, not only the death rate of HCC is high but also prognosis thereof is very poor. Currently, the efficacy of common chemotherapy drugs of HCC, for instance, Fluorouracil, Pirarubicin, Oxaliplatin, Cisplatin etc., is limited. The new targeted therapy drug Nexavar® (Sorafenib) as a multiple kinase inhibitors has been approved for advanced stage of HCC or primary HCC. Nexavar improves survival in HCC. In 2008, the global marketing of cancer treatment is $ 53.1 billion (Nature Review in Cancer) and the marketing of liver cancer treatment is around $ 2.5 billion. Therefore, development of new drugs for HCC treatment is unmet need in clinic.

### SUMMARY

One embodiment of the invention provides a pharmaceutical composition for promoting damaged liver regeneration, including: a proanthocyanidin with an effective amount; and a pharmaceutically acceptable carrier or salt, wherein the monomer units of the proanthocyanidin have the following formula:

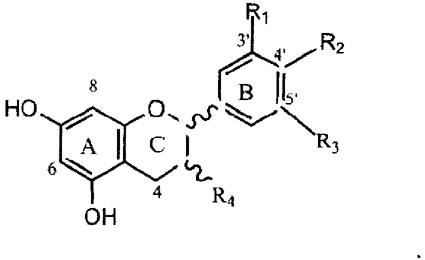

In the formula, when R₁ is OCH₃, R₂ is OH and R₃ is H, when R₁ is OH, R₂ is H and R₃ is H, when R₁ is OH, R₂ is OH and R₃ is H, or when R₁ is OH, R₂ is OH and R₃ is OH, and R₄ is 3-(α)-OH, 3-(β)-OH, 3-(α)-O-sugar or 3-(β)-O-sugar.

The pharmaceutical composition (BEL-X) is found after experimental tests that it can apply to the treatment of various liver diseases, including (1) liver cancer caused by chronic hepatitis B virus or hepatitis C virus infection. The pharmaceutical composition (BEL-X) can be used alone or used as adjuvant agents for other various treatment methods, which can improve the liver function of patients with HCC, reduced progression of HCC, increase the operable rate and success rate of surgery of patients with HCC, reduce the postoperative recurrent rate, increase the survival rate and prolong the survival time of HCC patients, furthermore, improve the quality of living of HCC patients. (2) The pharmaceutical composition (BEL-X) can be used alone or used in combination with other clinical treatment drugs to treat liver fibrosis patients. (3) The pharmaceutical composition (BEL-X) can be used alone or used in combination with other clinical treatment drugs to treat patients with fatty liver, as well as improve the liver functions to prevent liver cirrhosis and HCC.

Namely, the disclosure is as described as the items below.
1. A pharmaceutical composition for promoting damaged liver regeneration, comprising: a proanthocyanidin with an effective amount, the monomer unit of the proanthocyanidin having the following formula: 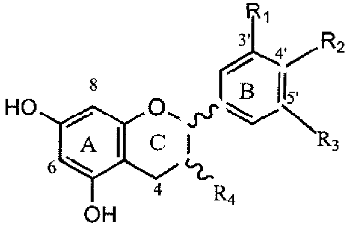
   wherein, when R₁ is OCH₃, R₂ is OH and R₃ is H, when R₁ is OH, R₂ is H and R₃ is H, when R₁ is OH, R₂ is OH and R₃ is H, or when R₁ is OH, R₂ is OH and R₃ is OH, and R₄ is 3-(α)-OH, 3-(β)-OH, 3-(α)-O-sugar or 3-(β)-O-sugar; and a pharmaceutically acceptable carrier or salt.
2. The pharmaceutical composition for promoting damaged liver regeneration as described in item 1, wherein the monomer units of the proanthocyanidin are bonded to each other via C4-C8 bonding, C4-C6 bonding, or C2-O7 bonding.
3. The pharmaceutical composition for promoting damaged liver regeneration as described in item 1, wherein the proanthocyanidin has a degree of polymerization ranging from 2 to 30.
4. The pharmaceutical composition for promoting damaged liver regeneration as described in item 1, wherein the monomer units of the proanthocyanidin comprise R or S optical isomers at C2, C3 or C4.
5. The pharmaceutical composition for promoting damaged liver regeneration as described in item 1, wherein the monomer units of the proanthocyanidin comprise flavonoid compounds.
6. The pharmaceutical composition for promoting damaged liver regeneration as described in item 5, wherein the flavonoid compounds comprise catechin, epicatechin, epiafzetechin, gallocatechin, galloepicatechin, epigallocatechin, flavonols, flavandiols, leucocyanidins or procynidins.
7. The pharmaceutical composition for promoting damaged liver regeneration as described in item 1, wherein the monomer unit of the proanthocyanidin comprises flavan-3-ol.
8. The pharmaceutical composition for promoting damaged liver regeneration as described in item 1, wherein the proanthocyanidin is extracted from a plant.
9. The pharmaceutical composition for promoting damaged liver regeneration as described in item 8, wherein the plant comprises Ericaceae, Rosaceae, Pinaceae, Vitaceae or Urticaceae plant.
10. The pharmaceutical composition for promoting damaged liver regeneration as described in item 9, wherein the Urticaceae plant comprises Boehmeria nivea L. Gaud.

A detailed description is given in the following embodiments with reference to the accompanying drawings.

### BRIEF DESCRIPTION OF THE DRAWINGS

The disclosure can be more fully understood by reading the subsequent detailed description and examples with references made to the accompanying drawing, wherein:

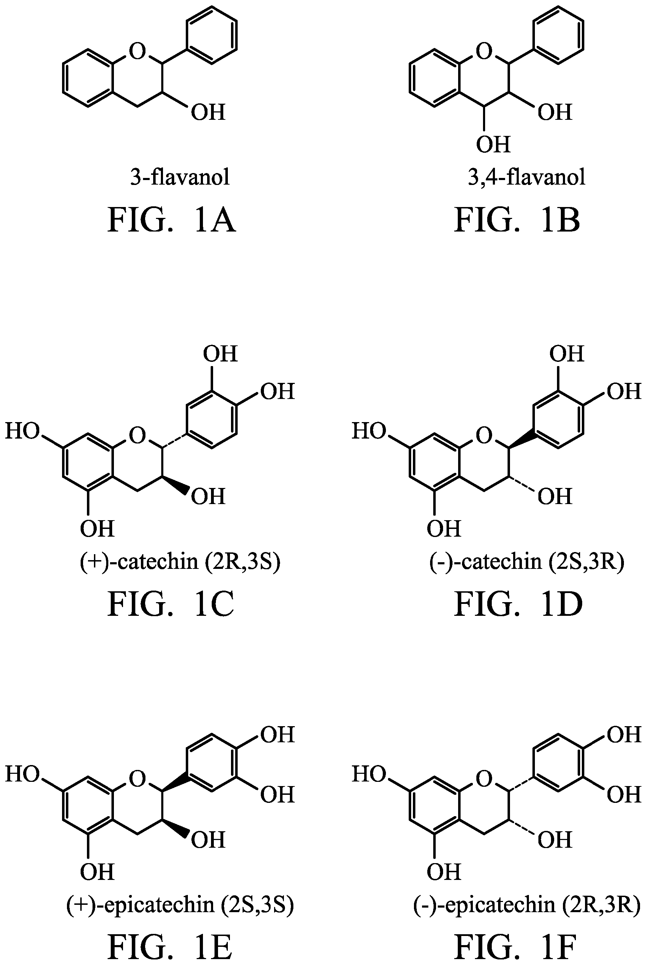

FIGS. 1a-1f represent 3-flavanol, 3,4-flavanol, catechin and epicatechin;
FIGS. 2a-2e represent pyrolysis gas chromatography mass spectrums of proanthocyanidin after re-purification of 95%-ethanol extract of proanthocyanidin Boehmeria nivea L. Gaud.;
FIG. 3 represents infrared absorption spectrum of proanthocyanidin after re-purification of 95%-ethanol extract of Boehmeria nivea L. Gaud.;
FIGS. 4a-4b represent of high performance liquid chromatography (+/-) mass spectrums of proanthocyanidin after re-purifcation of 95%-ethanol extract of Boehmeria nivea L. Gaud.;
FIGS. 5a-5c represent ¹³C NMR and ¹H NMR spectrums of proanthocyanidin after re-purification of 95%-ethanol extract of Boehmeria nivea L. Gaud.;
FIGS. 6a-6b respectively show C4-C8 bonding and C4-C6 bonding, in accordance with detection spectrums of ¹H NMR and ¹³C NMR, in the disclosure, monomer units of purified proanthocyanidin polymer are bonded to each other mainly via C4-C8 bonding;
FIGS. 7a-7c represent matrix assisted laser desorption ionization time-of-flight (MALDI-TOF) mass spectrums of proanthocyanidin after re-purification of 95%-ethanol extract of Boehmeria nivea L. Gaud.;
FIG. 8 shows effect of pharmaceutical compositions (BEL-X) on survival rate in hepatitis B virus X gene induced HCC transgenic mice, in the disclosure;
FIG. 9 shows effect of pharmaceutical compositions (BEL-X) on HCC in hepatitis B virus X gene induced HCC transgenic mice, in the disclosure, estimated by liver weight/body weight ratio;
FIG. 10 shows effect of pharmaceutical compositions (BEL-X) on liver function in hepatitis B virus X gene induced HCC transgenic mice, in the disclosure, estimated by liver function index - ALT;
FIG. 11 shows effect of pharmaceutical compositions (BEL-X) on liver function of hepatitis B virus X gene induced HCC transgenic mice, in the disclosure, estimated by liver function index - AST;
FIG. 12 shows effect of pharmaceutical compositions (BEL-X) on liver fibrosis induced by chemical substance DEN in rats, in the disclosure, estimated by hydroxyprolinc content;
FIG. 13 shows effect of pharmaceutical compositions (BEL-X) on liver fibrosis induced by chemical substance DEN in rats, in the disclosure, estimated by α-SMA staining area;
FIG. 14 shows effect of pharmaceutical compositions (BEL-X) on liver fibrosis induced by chemical substance DEN in rats, in the disclosure, estimated by hydroxyproline content;
FIGS. 15-16 show effect of pharmaceutical compositions (BEL-X) on survival rate of rats with liver fibrosis/liver cancer induced by chemical substance DEN, in the disclosure; and
FIG. 17 shows effect of pharmaceutical compositions (BEL-X) on liver regeneration of rats with liver fibrosis induced by chemical substance DEN, in the disclosure, estimated by regeneration ratio of liver volume.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The disclosure adopts proanthocyanidin as an active ingredient of the pharmaceutical composition (BEL-X) to achieve the purpose of promoting damaged liver regeneration.

In the disclosure, the proanthocyanidin having the effect of promoting damaged liver regeneration, may be extracted from a plant. In one embodiment, the used plant may comprise an Ericaceae, Rosaceae, Pinaceae, Vitaceae or Urticaceae plant, preferably Urticaceae Boehmeria nivea L. Gaud. The extracted part of the plant may comprise roots, stems, leaves and/or fruits.

In the disclosure, the plant may be extracted using general known methods. In one embodiment, dried roots, stems, leaves and/or fruits of a plant are sliced or grated. Next, the plant is extracted using an extraction solution. In one embodiment, roots and/or stems of the Boehmeria nivea L. Gaud. is selected for extract.

The extraction solution maybe selected from water or a solution mixed by water and solvents with different polarities from water. The solvents with different polarities from water may comprise alcohol, acetone, methanol or ethyl acetate. The solvents may be used alone, mixed with each other or mixed with water. The ratio of the extraction solution and the plant has no particular limitation. In one embodiment, the ratio of the extraction solution and the plant is 1:10 (W/W).

During the extraction, the extraction temperature may be slightly changed as different extraction solutions are selected. In one embodiment, the plant may be immersed in an extraction solution at room temperature. In another embodiment, the extraction solution may be heated to the reflux temperature (60-100°C) thereof. The extraction time is about 2 hours to seven days, depending on the extraction temperature. Additionally, during the extraction operation, for example, sodium chloride, dilute inorganic acid (e.g., dilute hydrochloric acid) or organic acid (e.g., vitamin C or tartaric acid), may be added to the extraction solution as needed to adjust the pH value of the extraction solution.

Next, the extract containing the proanthocyanidin active ingredient is concentrated and dried, or partial purification or complete purification may be performed on the extract as needed. In one embodiment, for the method of the partial purification, the dried extract is re-dissolved in 95% alcohol and/or a methanol aqueous solution. Next, the resulting solution is extracted using solvents with different polarities to remove partial impurities, for example, using an apolar solvent (e.g., n-hexane) to remove lipid and apolar substances, and then using trichloromethane and/or ethyl acetate to remove small phenol compounds. Next, the solvent-extracted liquid phase is concentrated and dried to obtain partially purified proanthocyanidin.

The complete purification method may comprise the following steps. The partially purified extract is dissolved in alcohol or methanol aqueous solution and placed into a molecular sieve column. Next, an elution is performed using different solutions and/or mixing solutions to purify and separate the proanthocyanidin. In one embodiment, the elution sequence of different solutions is 95% alcohol, 95% alcohol/methanol (1:1, v/v), 50% methanol and 50% acetone aqueous solution. The solutions which are eluted out through each eluent are collected in batches. Next, the purified proanthocyanidin in the eluted-out solution is detected using liquid chromatography (280nm). The proanthocyanidin solutions with various molecular weight distributions may be obtained by collecting the solutions which are eluted out through different eluents. Next, the eluted-out solution is concentrated at 40°C below and freeze-dried to obtain the purified proanthocyanidin. In one embodiment, the molecular sieve column used in the elution is Sephadex LH-20 column (purchased from German Amersham Corporation).

In the disclosure, the monomer units of the purified proanthocyanidin have the following formula.

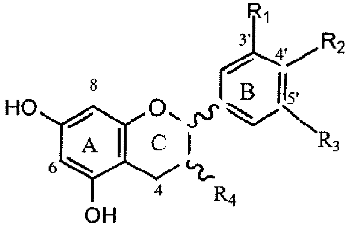

In one embodiment, when R₁ is OCH₃, R₂ is OH and R₃ is H. In another embodiment, when R₁ is OH, R₂ is H and R₃ is H. In another embodiment, when R₁ is OH, R₂ is OH and R₃ is H. In another embodiment, when R₁ is OH, R₂ is OH and R₃ is OH. In the formula, R₄ may be 3-(α)-OH, 3-(β)-OH, 3-(α)-O-sugar or 3-(β)-O-sugar.

The monomer units of the proanthocyanidin may comprise R or S optical isomers at C2, C3 or C4.

The structure of the monomer units of the proanthocyanidin may comprise flavonoid compounds, for example, catechin, epicatechin, epiafzetechin, gallocatechin, galloepicatechin, epigallocatechin, flavonols, flavandiols, leucocyanidins or procynidins. In one embodiment, the monomer units of the proanthocyanidin may comprise flavan-3-ol or flavane derivatives.

In the disclosure, the proanthocyanidin has a degree of polymerization ranging from 2 to 30, preferably from 3 to 20. The monomer units of the proanthocyanidin may be bonded to each other via C4-C8 bonding, C4-C6 bonding, or C2-O7 bonding. The proanthocyanidin has an average molecular weight ranging from 600 to 10,000.

In one embodiment, the purified proanthocyanidin may comprise proanthocyanidin with a single degree of polymerization. In another embodiment, the purified proanthocyanidin may comprise a proanthocyanidin mixture with various degrees of polymerization.

In the disclosure, the extracted proanthocyanidin may be prepared into a pharmaceutical composition for promoting damaged liver regeneration, which may comprise the proanthocyanidin and a pharmaceutically acceptable carrier or salt.

The pharmaceutically acceptable carrier may comprise, but is not limited to, a solvent, dispersion medium, coating, antibacterial agent, antifungal agent, isotonic absorption delaying agent or pharmaceutical compatibilizer. For different modes of administration, the pharmaceutical composition may be prepared into various suitable dosage forms using known methods.

The pharmaceutically acceptable salt may comprise, but is not limited to, inorganic salts or organic salts. The inorganic salts may comprise, for example, alkali metal salts such as sodium salts, potassium salts or amine salts, alkaline earth metal salts such as magnesium salts or calcium salts, or divalent or tetravalent cation salts such as zinc salts, aluminum salts or zirconium salts. The organic salts may comprise dicyclohexylamine salts, methyl-d-glucamine salts or amino acid salts such as arginine salts, lysine salts, histidine salts or glutamine salts.

The modes of administration of the pharmaceutical composition (BEL-X) may comprise oral, non-oral, through-inhalation-spray or through-implanted-reservoir administration. The non-oral modes may comprise subcutaneous, intracutaneous, intravenous, intramuscular, intraarticular, intraarterial, intrasynovial, intrasternal, intrathecal or intraleaional injection or perfusion techniques.

The oral dosage forms may comprise, but are not limited to, tablets, capsules, emulsions, aqueous suspensions, dispersions or solutions.

The pharmaceutical composition (BEL-X) is found after experimental tests that it can apply to the treatment of various liver diseases, including (1) liver cancer caused by infection from chronic hepatitis B virus or hepatitis C virus- The pharmaceutical composition (BEL-X) can be used alone or used as adjuvant agents for other various treatment methods, which can improve the liver functions of HCC patients , reduce progression of liver cancer, increase the operable rate and success rate of surgery of patients with HCC, reduce the postoperative recurrent rate, increase the survival rate and prolong the survival time of patients with HCC, as well as improve the quality of living of HCC patients. (2) The pharmaceutical composition (BEL-X) can be used alone or used in combination with other clinical drugs for liver fibrosis treatment. (3) The pharmaceutical composition (BEL-X) can be used alone or used in combination with other clinical drugs to treat patients with fatty liver, and improve the liver functions to prevent liver cirrhosis and liver cancer.

### Examples

### Example 1

### Structure determination of the monomer units of the proanthocyanidin polymer

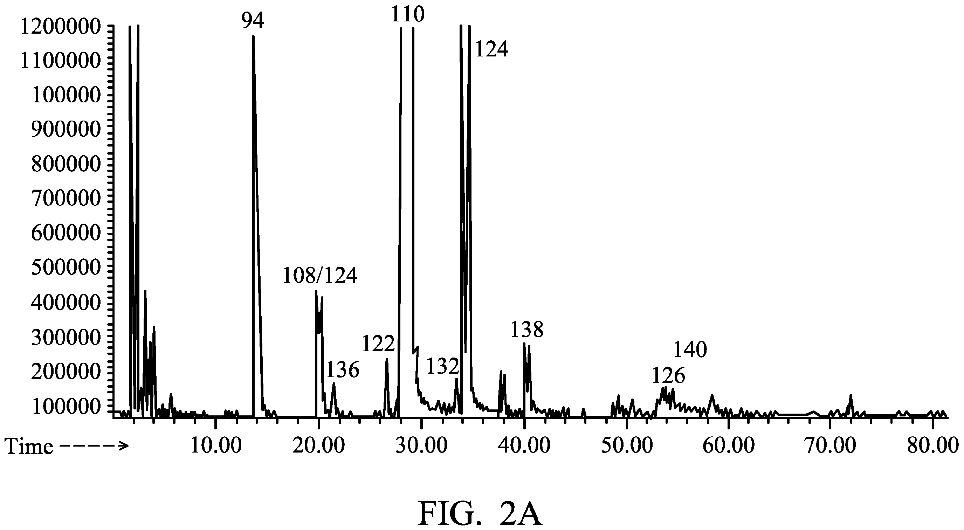

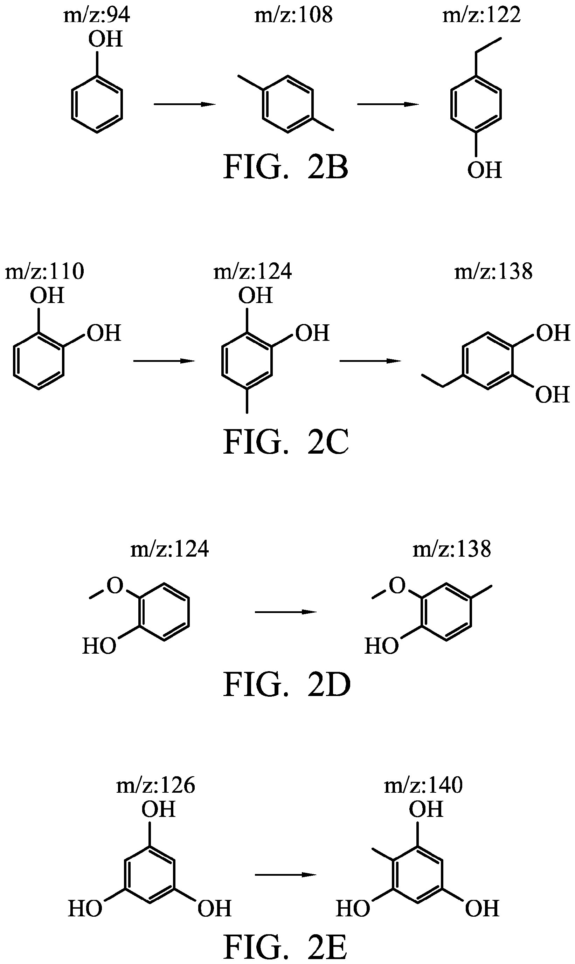

The monomer unit structure of the proanthocyanidin was detected by pyrolysis gas chromatography-mass spectrometry (PGC/MS). The detection method directly placed solid purified proanthocyanidin (a sample purified by the inventor) into pyrolysis gas chromatography and gradually warmed or instantaneously warmed with a segmented-temperature (50°C to 500°C) or single-temperature operation mode. The thermal-decomposed sample was separated through a specific metal column of the pyrolysis gas chromatography. The monomer unit structure of the proanthocyanidin polymer was determined by the spectrums produced from the detector of the mass spectrometry. The mass spectrum and structure analysis of the proanthocyanidin polymer are shown in FIGS 2a and 2b, respectively, wherein the left parts of FIGS. 2b-2e are the m/z values and chemical structures represented by the peaks in FIG. 2a, and the right parts thereof are the monomer unit analysis of the peaks in the left parts. The formula of the determined monomer unit structure of the proanthocyanidin polymer is represented as follows:

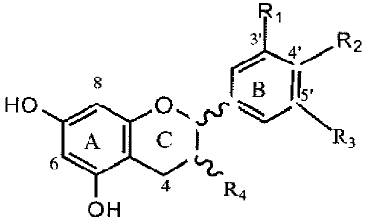

wherein, R₁ is OCH₃, R₂ is OH and R₃ is H, or R₁ is OH and R₂ and R₃ are H, or R₁ and R₂ are OH and R₃ is H, or R₁, R₂ and R₃ are OH.

The measured thermal-decomposed mass spectrums show the peaks of the glycoside signals. Therefore, it was presumed that R₄ may be 3-(α)-OH, 3-(β)-OH, 3-(α)-O-sugar or 3-(β)-O-sugar.

### Infrared absorption spectrum analysis

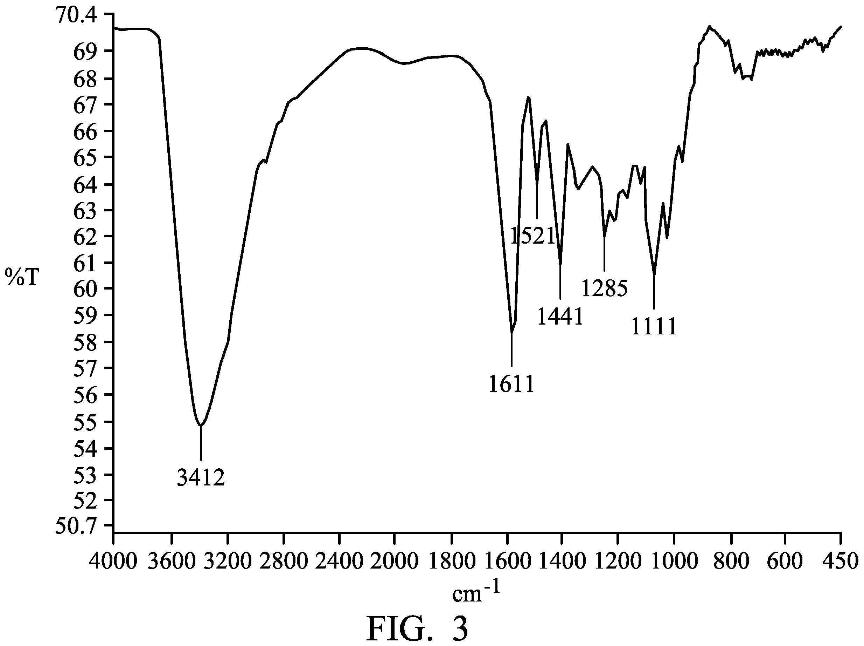

The purified proanthocyanidin sample and potassium chloride were mixed, tableted and detected by transmission infrared light. The result is shown in FIG. 3, wherein the strong absorption peaks were 3412.38nm, 1610.57nm, 1521.40nm, 1441.14nm, 1284.86 and 1100.88nm.

### Spectrum analysis of high performance liquid chromatography-mass spectrometry

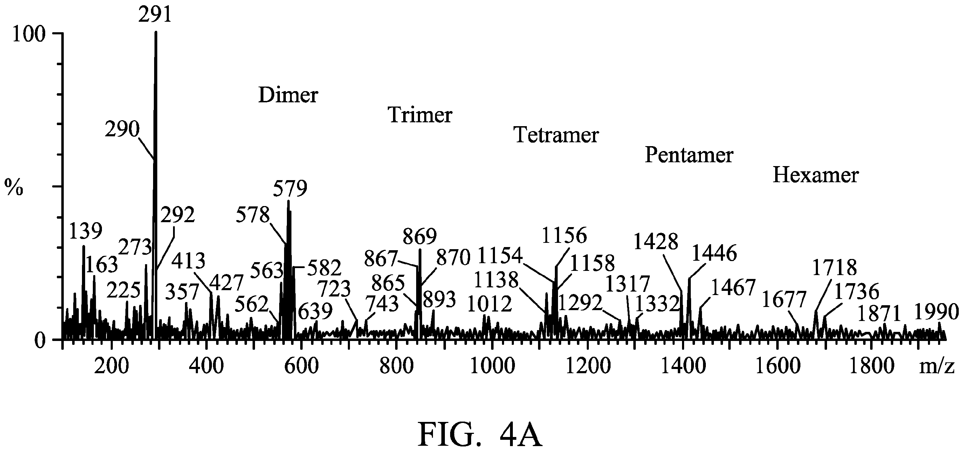

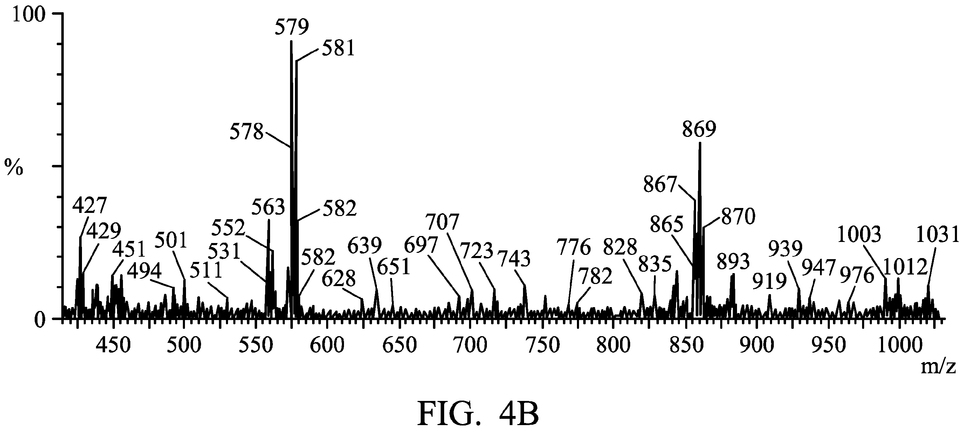

The purified proanthocyanidin sample was detected by high performance liquid chromatography-mass spectrometry (electrospray (+/-) mass spectrometry, HPLC/ESI+, HPLC/ESI-)(Micromass Quattro/Waters 2690). The monomer units and polymers of the proanthocyanidin with a degree of polymerization ranging from 1 to 6 and 164-glycoside (e.g., the molecular weights of the monomer units and molecular weight of 164 of a glycoside) were detected. The (+/-) mass spectrums of the high performance liquid chromatography-mass spectrometry of the purified proanthocyanidin are shown in FIGS. 4a and 4b.

### Spectrum analysis of ¹³C NMR and ¹H NMR

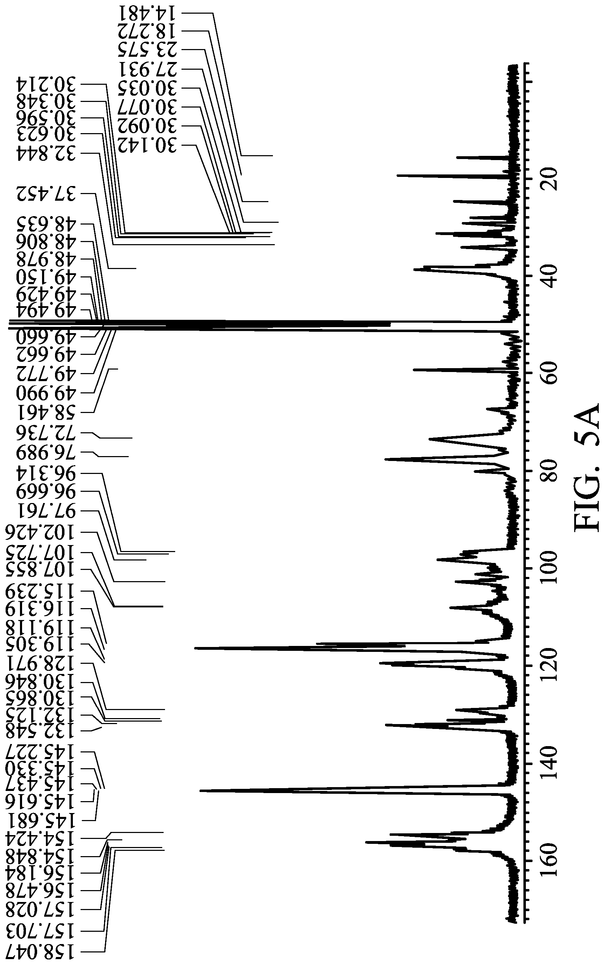

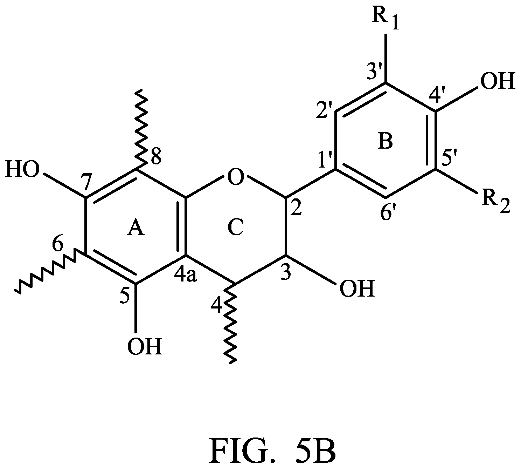

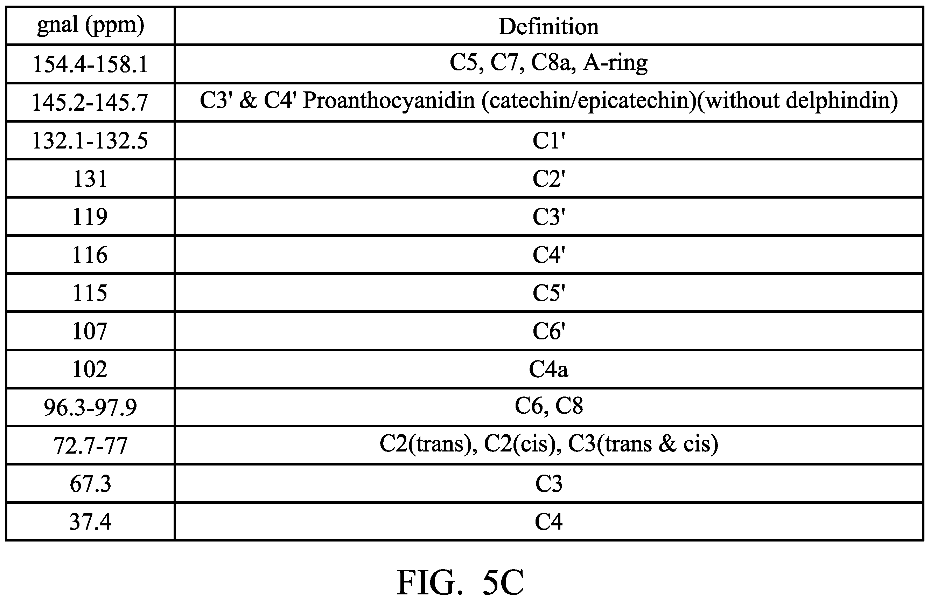

The purified proanthocyanidin sample was detected by ¹³C NMR and ¹H NMR. The detection results of ¹³C NMR are shown in FIGS. 5a-5c, wherein, at 142-145.7ppm, in addition to the doublet-doublet peaks, no other peaks are shown. The result indicated that the monomer units comprise anthocyanidin without delphindin (e.g., with three -OH groups in B ring thereof). The analysis results are the same as those of EGA/MS. In FIG. 5b, R₁=H or OH, R₂=H or OH or OCH₃.

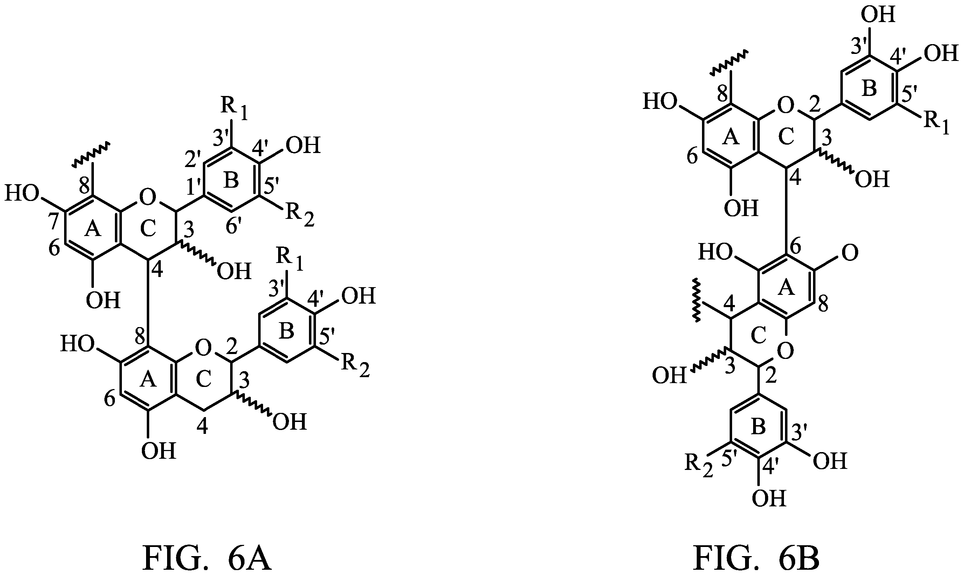

In accordance with the detection spectrums of ¹H NMR and ¹³C NMR, in the disclosure, the monomer units of the purified proanthocyanidin polymer are bonded to each other mainly via C4-C8 bonding. The C4-C8 bonding and C4-C6 bonding are shown in FIGS. 6a and 6b, respectively.

### Spectrum analysis of matrix assisted laser desorption ionization time-of-flight (MALDI-TOF) mass spectrometry

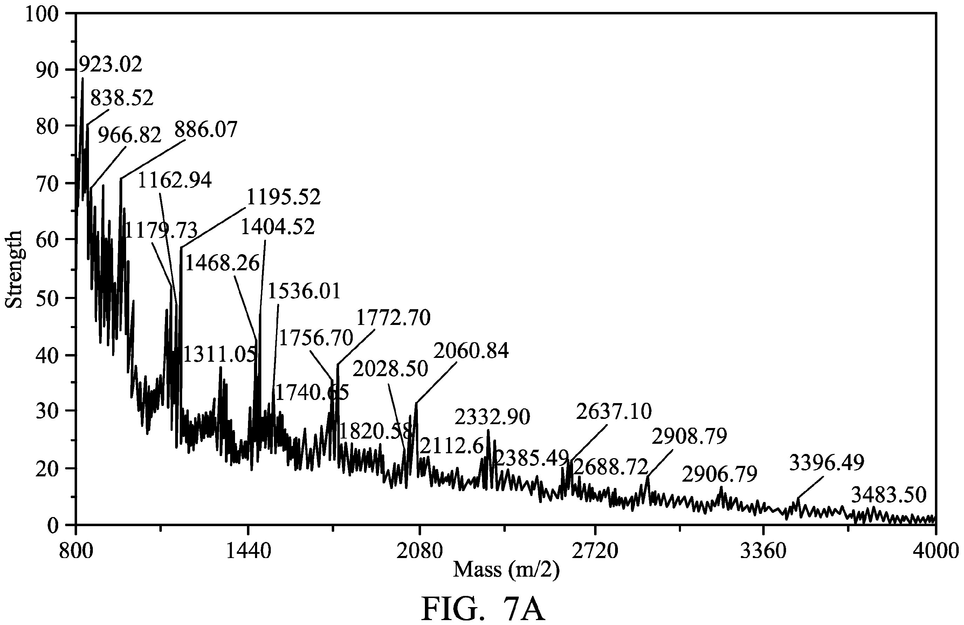

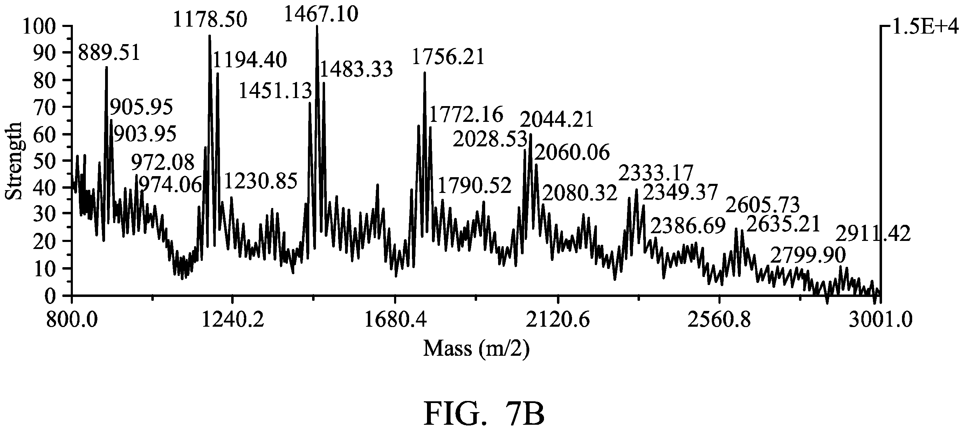

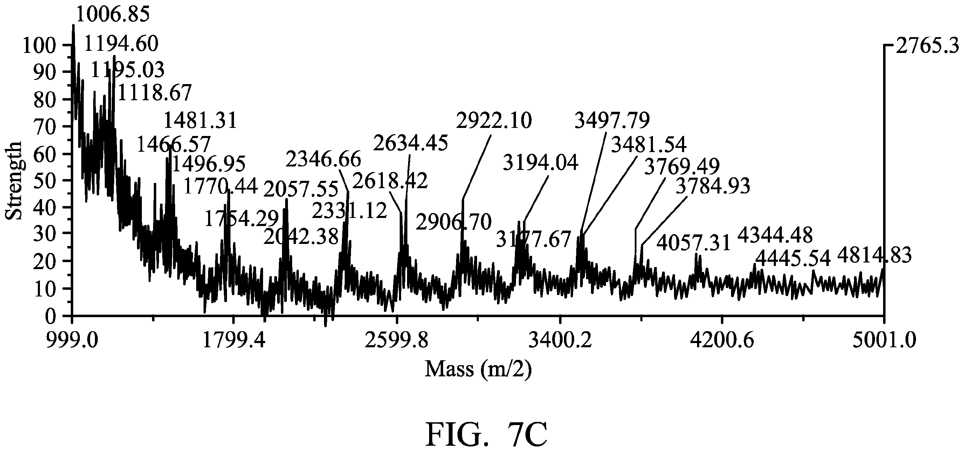

The molecular weight distribution of the partially purified proanthocyanidin was detected by a matrix assisted laser desorption ionization time-of-flight (MALDI-TOF) mass spectrometry. The results are shown in FIGS. 7a-7c. The detection results indicated that the molecular weight distribution of the partially purified proanthocyanidin was from 500 to 5,000. It was presumed that the polymer had a degree of polymerization ranging from 2 to 18 in accordance with the detection results of the molecular weight distribution.

### Example 2

### Preparation of the proanthocyanidin-containing extract (1)

Roots and stems connecting the roots of the Boehmeria nivea L. Gaud. medicinal material were washed with water and dried under a natural environment. The dried medicinal material was cut into slices having a thickness of about 5mm and stored at 4°C. The stored Boehmeria nivea L. Gaud. medicinal material was ground by a muller and passed through a 20-mesh sieve. The obtained powder was dispersed in 95% ethanol (10 times by weight)(1:10, w/w) and thermal-refluxed for 2 hours (twice). After cooled to room temperature, the extracting solution was collected. The extracting solution was centrifuged and filtrated by a centrifuge. The filtrate was then concentrated by a reduced-pressure concentrator at a temperature below 40°C and dried by a freeze-dryer to obtain a proanthocyanidin-containing extract.

### Example 3

### Preparation of the proanthocyanidin-containing extract (2)

The dried medicinal material stored at 4°C of Example 2 was ground by a muller and passed through a 20-mesh sieve. The obtained powder was dispersed in reverse osmosis water (10 times by weighs)(1:10, w/w) and thermal-refluxed for 2 hours (twice). After cooled to room temperature, the extracting solution was collected. 50%-95% ethanol was added to the extracting solution and cooled to cause precipitation. The supernatant was centrifuged and filtrated by a centrifuge. The filtrate was then concentrated by a reduced-pressure concentrator at a temperature below 40°C and dried by a freeze-dryer to obtain a proanthocyanidin-containing extract.

### Example 4

### Purification of the proanthocyanidin-containing extract (1)

The proanthocyanidin-containing extract of Example 2 or 3 was added to n-hexane (1:10, w/v) and thermal-refluxed (by Soxhelt apparatus) for 6 hours to remove lipid in the extract. The obtained solid was dissolved in a 70% methanol aqueous solution and/or 0.3% vitamin C aqueous solution and concentrated by a reduced-pressure concentrator at a temperature below 40°C to remove solvent. The concentrates were then added to trichloromethane (trichloromethane: concentrates=1:1, v/v) and oscillated for 30 minutes by an oscillator (multi-extraction). Ethyl acetate was added to the liquid phase (ethyl acetate: liquid phase-1:1. v/v) and oscillated for 30 minutes (multi-extraction). The liquid phase was then concentrated by a reduced-pressure concentrator at a temperature below 40°C and dried by a freeze-dryer to obtain partially purified proanthocyanidin.

### Example 5

### Purification of the proanthocyanidin-containing extract (2)

The proanthocyanidin-containing extract of Example 2 or 3 was dissolved in water/ethanol (1:10, w/v). Next, n-hexane was added (1:10, v/v) and oscillated for 30 minutes by an oscillator (multi-extraction) to remove lipid in the extract. Ethyl acetate was added to the liquid phase (ethyl acetate: liquid phase=1:1, v/v) and oscillated for 30 minutes (multi-extraction). The liquid phase was added to n-butanol (1:10, v/v) and oscillated for 30 minutes by an oscillator (multi-extraction). The liquid phase was then concentrated by a reduced-pressure concentrator at a temperature below 40°C and dried by a freeze-dryer to obtain partially purified proanthocyanidin.

### Example 6

### Purification of the proanthocyanidin-containing extract (3)

The partially purified proanthocyanidin obtained from Example 4 was repurified by molecular sieve column chromatography (gel permeation chromatography column, Sephadex LH-20, 4cm diameter x 45cm long). First, an elution was performed using solutions with different polarities to remove impurities. Next, 2.5g of partially purified proanthocyanidin was dissolved in 0.5ml 95% ethanol. The dissolved sample was then placed into a molecular sieve column and continuously eluted out by a series of solvents (eluents). The eluates eluted out through various solvents (eluents) were collected. The eluents were 300ml 95% ethanol, 300ml 95% ethanol/methanol (1/1, v/v), 300ml methanol. 300ml of a 50% methanol aqueous solution and 300ml of a 50% acetone aqueous solution, sequentially. Except for the eluate eluted out through the eluent of 300ml 95% ethanol, other eluted-out eluates were then concentrated by a reduced-pressure concentrator at a temperature below 40°C and dried by a freeze-dryer to obtain partially purified or completely purified proanthocyanidin. The dried substance was then stored at -20°C. Only the examples corresponding to the present claims, i.e. to the promotion of damaged liver regeneration (examples 14,15) are part of the invention.

### Example 7

### Effect of drugs (BEL-X) on survival rate of liver cancer-induced hepatitis B virus X transgenic mice

Experimental animals: The parental provenance of the animals used by the experiment was male hepatitis B virus X gene transgenic mice (C57BL/6J-HBx (A0112 line)) published by BBRC¹ 2006.

Experimental grouping and experimental design: The mice were divided into 6 groups, including one control group of non-transgenic mice (Non-Tg mock 9-20M), one drug control group of non-transgenic mice (Non-Tg BEL-X treated 9-20M), one control group of transgenic mice (Tg mock 9-20M), and three drug testing groups of transgenic mice (Tg BEL-X treated): administration of an oral drug BEL-X (the pharmaceutical composition of the disclosure) to the mice once daily, respectively, from the age of 9 months to 20 months (Tg BEL-X treated 9-20M), from the age of 12 months to 20 months (Tg BEL-X treated 12-20M) and from the age of 15 months to 20 months (Tg BEL-X treated 15-20M). In the control group of the non-transgenic mice (Non-Tg mock 9-20M) and the control group of the transgenic mice (Tg mock 9-20M), a drinking water was given to the mice once daily from the age of 9 months to 20 months. In the drug control group of the non-transgenic mice (Non-Tg BEL-X treated 9-20M), an oral drug BEL-X was administrated to the mice once daily from the age of 9 months to 20 months. The dosage of the BEL-X drug was 1,000mg/kg/day.

### Conclusion:

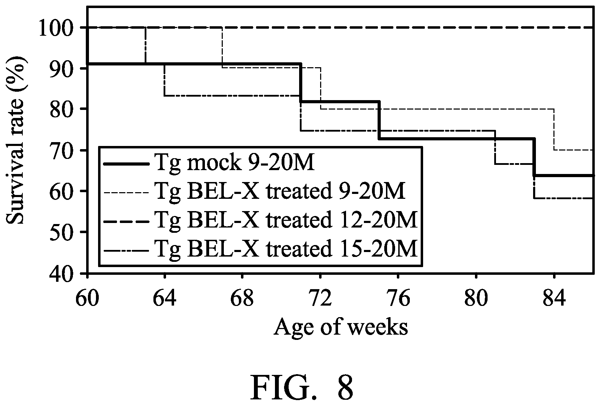

1. Referring to FIG. 8, 100% of the male hepatitis B virus X gene transgenic mice produced liver cancer at the age of 20 months, and the survival rate thereof was about 64% (Tg mock 9-20M). The survival rate of the mice fed with the drug BEL-X at various ages was, respectively, 70% (the age of 9-20 months, Tg BEL-X treated 9-20M), 100% (the age of 12-20 months, Tg BEL-X treated 12-20M) and 58% (the age of 15-20 months, Tg BEL-X treated 15-20M).
2. By Chi-Square statistical analysis, the survival rate of the male hepatitis B virus X gene transgenic mice fed with the drug BEL-X at the age of 12-20 months was 100% at the age of 20 months, significantly.
3. Feeding the male hepatitis B virus X gene transgenic mice with the BEL-X in an early stage can improve survival rate.

### Example 8

### Effect of drugs (BEL-X) on retardation of HCC in hepatitis B virus X gene transgenic mice

Experimental animals: The parental provenance of the animals used by the experiment was male hepatitis B virus X gene transgenic mice (C57BL/6J-HBx (A0112 line)) published by BBRC¹ 2006.

Experimental grouping and experimental design: The mice were divided into 6 groups, including one control group of non-transgenic mice (Non-Tg mock 9-20M), one drug control group of non-transgenic mice (Non-Tg BEL-X treated 9-20M), one control group of transgenic mice (Tg mock 9-20M), and three drug experimental groups of transgenic mice (Tg BEL-X treated): administration of an oral drug BEL-X (the pharmaceutical composition of the disclosure) to the mice once daily, respectively, from the age of 9 months to 20 months (Tg BEL-X treated 9-20M), from the age of 12 months to 20 months (Tg BEL-X treated 12-20M) and from the age of 15 months to 20 months (Tg BEL-X treated 15-20M). In the control group of the non-transgenic mice (Non-Tg mock 9-20M) and the control group of the transgenic mice (Tg mock 9-20M), a drinking water was given to the mice once daily from the age of 9 months to 20 months. In the drug control group of the non-transgenic mice (Non-Tg BEL-X treated 9-20M), an oral drug BEL-X was administrated to the mice once daily from the age of 9 months to 20 months. The dosage of the BEL-X drug was 1,000mg/kg/day.

Determination of the ratio of liver weight and body weight: The animals were sacrificed and dissected for liver (containing liver tumors) sampling. The scaled liver weight was divided by the body weight of the mice to obtain the ratio of the liver weight and the body weight.

### Conclusion:

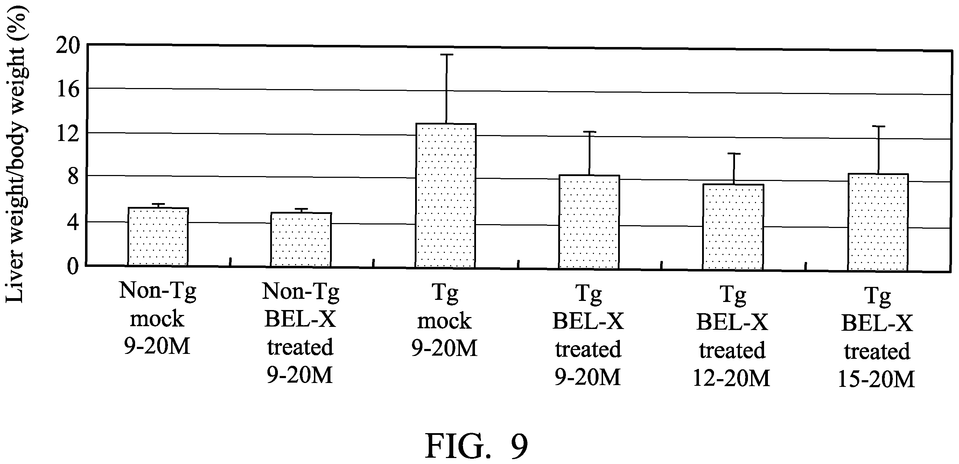

1. Referring to FIG. 9, the ratio of the liver weight and the body weight of the normal non-transgenic mice (Non-Tg mock) was about 5%. The ratio of the liver weight and the body weight of the male hepatitis B virus X gene transgenic mice (Tg mock) increased to about 13% due to production of liver cancer at the age of 20 months. By ANOVA statistical analysis, the variation of the ratio of the liver weight and the body weight of the transgenic mice and the normal non-transgenic mice was significant.
2. After feeding the normal non-transgenic mice with the BEL-X for one year (the age of 9-20 months, Non-Tg BEL-X treated 9-20M), the ratio of the liver weight and the body weight was 5% the same as that of the no-drug-feeding groups. The result indicated that there was no any effect of the drug on normal animals.
3. Feeding the male hepatitis B virus X gene transgenic mice with the drug BEL-X at various ages resulted in that the ratio of the liver weight and the body weight was reduced to about 8% in the three groups. The variation of the ratio of the liver weight and the body weight of the drug-feeding groups of the mice with the age of 9-20 months (Tg BEL-X treated 9-20M) and the mice with the age of 12-20 months (Tg BEL-X treated 12-20M) and the no-drug-feeding group (Tg mock 9-20M) was statistically significant.

### Example 9

### Effect of drugs (BEL-X) on liver functions in hepatitis B virus X gene induced HCC transgenic mice (1)

Experimental animals: The parental provenance of the animals used by the experiment was male hepatitis B virus X gene transgenic mice (C57BL/6J-HBx (A0112 line)) published by BBRC¹ 2006.

Experimental grouping and experimental design: The mice were divided into 6 groups, including one control group of non-transgenic mice (Non-Tg mock 9-18M), one drug control group of non-transgenic mice (Non-Tg BEL-X treated 9-18M), one control group of transgenic mice (Tg mock 9-18M), and three drug experimental groups of transgenic mice (Tg BEL-X treated): administration of an oral drug BEL-X (the pharmaceutical composition of the disclosure) to the mice once daily, respectively, from the age of 9 months to 18 months (Tg BEL-X treated 9-18M), from the age of 12 months to 18 months (Tg BEL-X treated 12-18M) and from the age of 15 months to 18 months (Tg BEL-X treated 15-18M). In the control group of the non-transgenic mice (Non-Tg mock 9-18M) and the control group of the transgenic mice (Tg mock 9-18M), a drinking water was given to the mice once daily from the age of 9 months to 18 months. In the drug control group of the non-transgenic mice (Non-Tg BEL-X treated 9-18M), an oral drug BEL-X was administrated to the mice once daily from the age of 9 months to 18 months. The dosage of the BEL-X drug was 1,000mg/kg/day.

Detection of the liver function - ICG: The mice were intravenously injected with indocyanine green (ICG). After 10 minutes, the concentration (mg/dl) of ICG remaining in the blood was detected, as an index of liver function. This experiment was carried out 2 times, respectively, at the age of 12 months and 18 months of the mice.

### Conclusion:

1. Referring to the following Table 1, the ICG metabolic value of the normal non-transgenic mice at the age of 18 months (Non-Tg mock 9-18M) was 2.25 ±0.89mg/dl. There was no significant difference between this result and that of the group fed with the BEL-X at the age of 18 months (Non-Tg BEL-X treated 9-18M).
2. The ICG metabolism of the male hepatitis B virus X gene transgenic mice (Tg mock 9-18M) slowed down and the value increased to 4.46±1.17mg/dl due to production of liver cancer at the age of 18 months. By nonparametric statistical analysis, the difference of the ICG metabolism of the transgenic mice and the normal non-transgenic mice was significant.
3. Feeding the male hepatitis B virus X gene transgenic mice with the drug BEL-X at various ages in the three groups resulted in that the ICG metabolic values of the three groups were lower than that of the no-drug-feeding group (Tg mock 9-18M). The difference of the ICG metabolic value of the group fed with the drug BEL-X from the age of 9 months (Tg BEL-X treated 9-18M) and that of the no-drug-feeding group (Tg mock 9-18M) was statistically significant. The result indicated that the BEL-X can improve the liver functions of HCC animals.

**Table 1**

| Groups | ICG concentration (mg/dl) in blood at the age of 18 months |
|---|---|
| Non-Tg mock 9-18M | 2.25±0.89 |
| Non-Tg BEL-X treated 9-18M | 2.13±0.92 |
| Tg mock 9-18M | 4.46±1.17 |
| Tg BEL-X treated 9-18M | 2.63±0.76 |
| Tg BEL-X treated 12-18M | 3,47±0.77 |
| Tg BEL-X treated 15-18M | 3.87±0.72 |

### Example 10

### Effect of drugs (BEL-X) on liver functions in hepatitis B virus X gene induced HCC transgenic mice (2)

Experimental animals: The parental provenance of the animals used by the experiment was male hepatitis B virus X gene transgenic mice (C57BLI6J-HBx (A0112 line)) published by BBRC¹ 2006.

Experimental grouping and experimental design: The mice were divided into 6 groups, including one control group of non-transgenic mice (Non-Tg mock 9-20M), one drug control group of non-transgenic mice (Non-Tg BEL-X treated 9-20M), one control group of transgenic mice (Tg mock 9-20M), and three drug experimental groups of transgenic mice (Tg BEL-X treated): administration of an oral drug BEL-X (the pharmaceutical composition of the disclosure) to the mice once daily, respectively, from the age of 9 months to 20 months (Tg BEL-X treated 9-20M), from the age of 12 months to 20 months (Tg BEL-X treated 12-20M) and from the age of 15 months to 20 months (Tg BEL-X treated 15-20M). In the control group of the non-transgenic mice (Non-Tg mock 9-20M) and the control group of the transgenic mice (Tg mock 9-20M), a drinking water was given to the mice once daily from the age of 9 months to 20 months. In the drug control group of the non-transgenic mice (Non-Tg BEL-X treated 9-20M), an oral drug BEL-X was administrated to the mice once daily from the age of 9 months to 20 months. The dosage of the BEL-X drug was 1,000mg/kg/day.

Detection of the liver functions - alanine aminotransferase (ALT) and aspartate aminotransferase (AST): All mice were blooded (from jaw or heart) once regular monthly. Whole blood was stood in the eppendorf at room temperature for more than 30 minutes. After coagulation, the blood sample was centrifuged with 1,800xg for 10 minutes. After centrifugation, the serum was removed into new eppendorf and stored at -20°C until the day of testing. The ALT and AST values of the serum were determined by a wet serum biochemical analyzer (HITACHI 7080). From the age of 9 months, the measured liver function indexes (ALT and AST) of the mice at the age of 9-20 months of each group were comprehensively analyzed every 3 months due to the correlation between the liver lesions and the age of the male hepatitis B virus X gene transgenic mice.

### Conclusion:

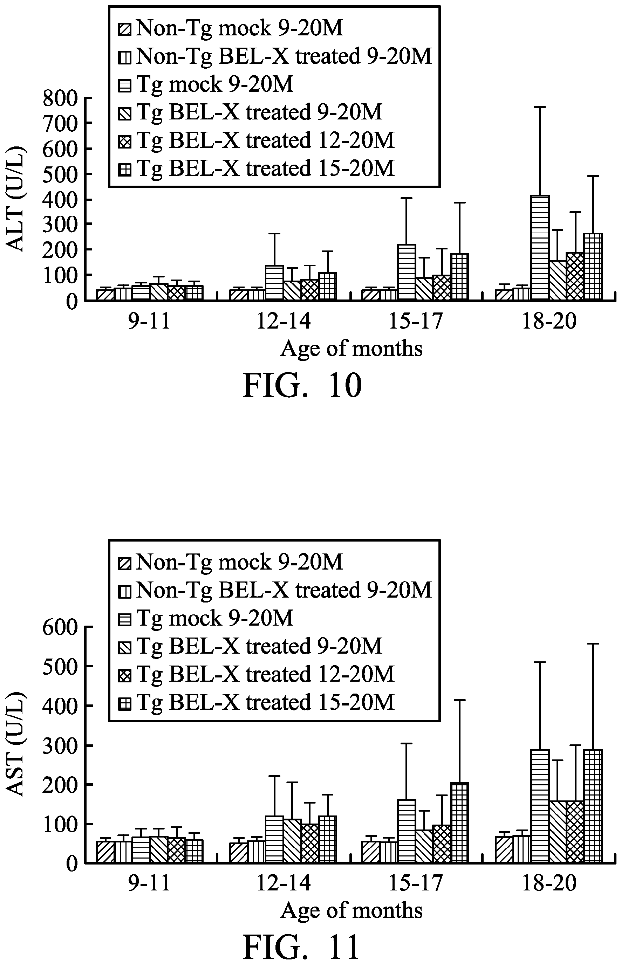

1. Referring to FIGS. 10 and 11, the difference of ALT and AST of the normal non-transgenic mice (Non-Tg mock 9-20M) and the hepatitis B virus X gene transgenic mice (Tg mock 9-20M) occurred from the age of 12 months. There was no significant difference of the liver function indexes between the group of the normal non-transgenic mice fed with the BEL-X (Non-Tg BEL-X treated 9-20M) and the no-drug-feeding group (Non-Tg mock 9-20M).
2. Feeding the hepatitis B virus X gene transgenic mice with the drug BEL-X at various ages resulted in that the ALT and AST of the three groups were lower than that of the no-drug-feeding group (Tg mock 9-18M). The difference of the ALT and AST of the drug-feeding groups of the age of 9-20 months (Tg BEL-X treated 9-20M) and the age of 12-20 months (Tg BEL-X treated 12-20M) and that of the no-drug-feeding group (Tg mock 9-20M) was statistically significant. The result indicated that the BEL-X can effectively improve the liver functions of HCC animals.

### Example 11

### Effect of drugs (BEL-X) on liver fibrosis induced by chemical substance DEN of rats (1)

Experimental grouping and experimental design: 8-week-old Wistar rats were fed with diethyl nitrosamine (DEN)(100ppm, added in water) for 6 weeks (D6 group) and 9 weeks (D9 group) to induce liver fibrosis and liver cancer. In the other two groups, the rats were simultaneously fed with DEN and the BEL-X drug (1000mg/kg body weight)(added in feed and fed every day for 6 weeks (D6H6 group) and 9 weeks (D9H9 group)). Degree of liver cancer of the rats was analyzed at various time points. In the control groups, no any drug was administrated during the entire process. Each experimental group had 10 rats. After pathological section and staining, the degree of liver fibrosis/liver cancer was interpreted, assisting with hydroxyproline biochemical analysis. An increase of hydroxyproline content in liver was used as an index of liver fibrosis. Livers of rats of each group were collected at various time points to determine the hydroxyproline content. Next, the livers were sectioned and α-SMA-immunostaining analysis was carried out. An increase of α-smooth-muscle-actin (α-SMA) content was also used as another index of liver fibrosis. At the 9^{th} week, livers of rats of each group were collected and α-SMA-immunostaining was carried out. Liver cells were observed using a microscope and the amount of cells containing the label was calculated.

### Conclusion:

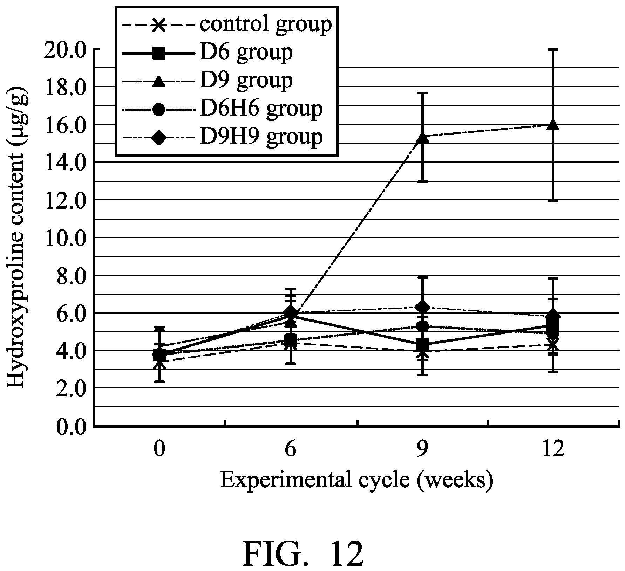

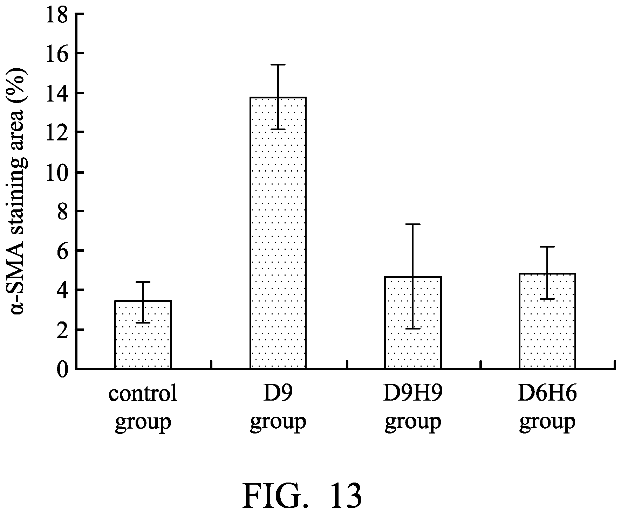

1. Referring to FIG. 12, in the group where DEN was continuously fed for 9 weeks (D9 group), the hydroxyproline content in livers significantly increased. The result indicated that DEN induced liver fibrosis. However, in the experimental group where BEL-X (D9H9 group) was continuously fed, the hydroxyproline content significantly decreased. The result indicated that BEL-X prevented liver from fibrosis caused by the chemical substance DEN.
2. Referring to FIG. 13, in the group where DEN was continuously fed for 9 weeks (D9 group), the α-smooth-muscle-actin (α-SMA) content in livers significantly increased. The result indicated that DEN induced liver fibrosis. However, in the experimental group where BEL-X (D9H9 group) was continuously fed, the α-smooth-muscle-actin (α-SMA) content significantly decreased. The result indicated that BEL-X prevented liver from fibrosis caused by the chemical substance DEN.
3. The α-smooth-muscle-actin (α-SMA) content also significantly decreased in the experimental group where DEN was continuously fed and BEL-X for 6 weeks (D6H6 group). The result indicated that BEL-X prevented liver in carly stage from fibrosis caused by the chemical substance DEN.

### Example 12

### Effect of drugs (BEL-X) on liver fibrosis induced by chemical substance DEN of rats (2)

Experimental grouping and experimental design: 8-week-old Wistar rats were fed with diethyl nitrosamine (DEN)(100ppm, added in water) to induce liver fibrosis and liver cancer. In other three groups, the rats were simultaneously fed with DEN and the BEL-X drug (1000mg/kg body weight). The BEL-X was fed in 3 different groups from the 3^{rd} to 6^{th} week (DEN-BEL-X 3-6), from the 6^{th} to 9^{th} week (DEN-BEL-X 6-9) and from the 9^{th} to 12^{th} week (DEN-BEL-X 9-12), respectively. The degree of liver cancer of the rats was analyzed at proper time points. In the control group (DEN), no drug was administrated during the entire process of feeding the DEN. Each experimental group had 10 rats. The degree of liver fibrosis/liver cancer was interpreted via a visual method, assisting with hydroxyproline biochemical analysis. An increase of hydroxyproline content in liver was used as an index of liver fibrosis. Livers of rats of each group were collected at the 12^{th} week to determine the hydroxyproline content.

### Conclusion:

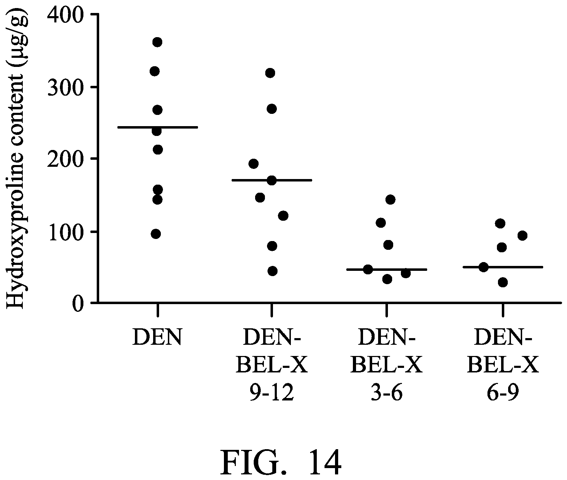

1. Referring to FIG. 14, in the untreated group (DEN) where DEN was continuously fed for 9 weeks, the hydroxyproline content in livers significantly increased. The result indicated that DEN induced liver fibrosis. In contrast, the hydroxyproline content was significantly decreased in the early stages of feeding for the BEL-X groups (DEN-BEL-X 3-6 and DEN-BEL-X 6-9) The results demonstrate that BEL-X reversed liver fibrosis caused by the chemical substance DEN.

### Example 13

### Effect of drugs (BEL-X) on survival rate of rats with liver fibrosis induced by chemical substance DEN

Experimental grouping and experimental design: 8-week-old Wistar rats were fed with diethyl nitrosamine (DEN)(50ppm, added in water) for 10.5 weeks to induce liver fibrosis and liver cancer (B group). The rats were simultaneously fed with DEN and the BEL-X drug (1000mg/kg body weight)(added in feed and fed every day, respectively, from the 0^{th} to 10.5^{th} week (C group), from the 3^{rd} to 10.5^{th} week (D group), and from the 6^{th} to 10.5^{th} week (E group). The BEL-X was fed for 3 weeks after stopping feeding the DEN (F group). The degree of liver cancer of the rats was analyzed at proper time points. In the control group (A group), no drug was administrated during the entire process. Animal deaths were recorded during the experiment. The survival rate of each group was analyzed by nonparametric statistics.

### Conclusion:

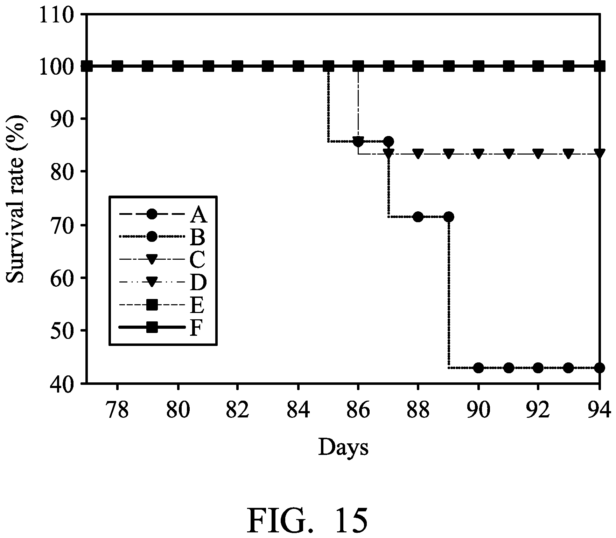

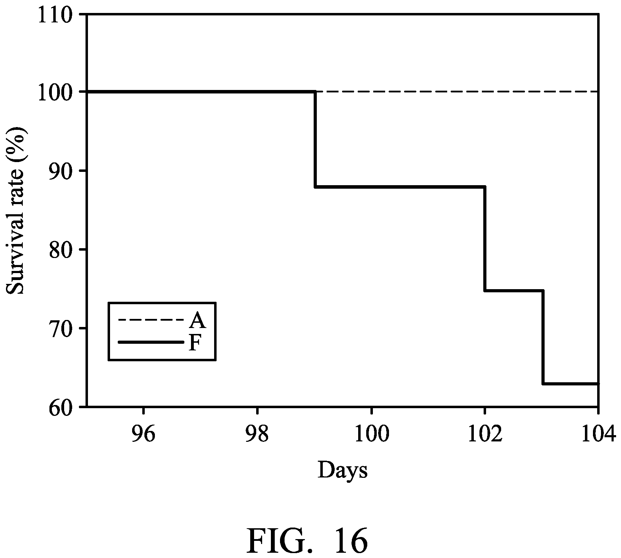

1. Referring to FIG. 15, analyzing the survival rate of each group at the 13.5^{th} week (94^{th} day) resulted in that, in the B group where only DEN was fed, the survival rate was merely about 40%. In contrast, the survival rates of feeding BEL-X at various periods were over 80%, The results indicated that BEL-X improved the survival rate of rats with liver fibrosis and liver cancer effectively.
2. Referring to FIG. 16, analyzing the survival rate of the rats administrated with the BEL-X drug for 3 weeks after inducing liver cancer by DEN (F group) at the 15^{th} week (104^{th} day) resulted in that, the survival rate of rats was 100% in the period (74^{th}-94^{th} day) of administrating BEL-X. Furthermore, no mortality was found during the five days (95^{th}-99^{th} day) after stopping the administration of the BEL-X drug.. The survival rate at 15^{th} week (104^{th} day) was 62% which is still higher than the survival rate of 40% at the 13.5^{th} week (94^{th} day) of B group where only DEN was fed. The results indicated that BEL-X can prolong the survival time as well as improve the survival rate of the rats with liver cirrhosis and liver cancer effectively.

### Example 14

### Effect of drugs (BEL-X) on regeneration of DEN damaged liver after hepatectomy (1)

Experimental grouping and experimental design: 8-week-old Wistar rats were fed with diethyl nitrosamine (DEN)(100ppm, added in water) for 9 weeks to induce liver fibrosis and liver cancer (no-drug-feeding group). The treated rats were simultaneously fed with the BEL-X drug (divided into high-dosage BEL-X group (1000mg/kg body weight) and low-dosage BEL-X group (250mg/kg body weight)) from 6^{th} to 9^{th} week. After feeding of the drug was completed, 50% of liver lobes were resected at the 9^{th} week. After two days, the liver sample was collected and sectioned, and H&E staining was carried out. Next, the mitosis of liver cells was observed under a microscope, as a basis for liver regeneration. The mitosis of liver cells was calculated as follows. Each rat provided at least three liver slices. Each slice comprised 10 fields. The number of mitotic cells was counted under a 400X magnification microscope. Finally, the average value of the number of mitotic cells of rats of each group was obtained.

### Conclusion:

1. Referring to the following Table 2, performing hepatectomy after inducing liver fibrosis and liver cancer with the chemical substance DEN resulted in that the mitosis count (7.6±4.6) of the livers of the rats of the no-drug-feeding group was much more lower than the mitosis count (12±5.5 or 13.0±5.6) of the livers of the rats simultaneously fed with high-dosage or low-dosage BEL-X drug for 3 weeks. The results indicated that, the liver regeneration can be improved in the chemical DEN damaged liver effectively.

**Table 2**

| Groups | Mitosis counts |
|---|---|
| High-dosage BBL-X group | 12.0±5.5 |
| Low-dosage BEL-X group | 13.0±5.6 |
| No-drug-feeding group | 7.6±4.6 |

### Example 15

### Effect of drugs (BEL-X) on regeneration of DEN damaged liver after hepatectomy (2)

Experimental grouping and experimental design: 8-week-old Wistar rats were fed with diethyl nitrosamine (DEN)(100ppm, added in water) for 9 weeks to induce liver fibrosis and liver cirrhosis (DEN group). The treated rats were simultaneously fed with the BEL-X drug (1000mg/kg body weight) from the 6^{th} to 9^{th} week (BEL-X group). No administration of both DEN and BEL-X rats were as the control group. After feeding of the drug was completed, a magnetic resonance imaging examination was carried out and 30% of liver lobes were resected at the 9^{th} week. In the control group not fed with DEN and BEL-X, the same surgery was performed. After two weeks, the second magnetic resonance imaging examination was carried out and the rats were sacrificed. The animal deaths were recorded during the experiment. After the surgery, the eating time and feeding amount of rats of each group were observed. The survival rate of the rats of each group was calculated.

### Conclusion:

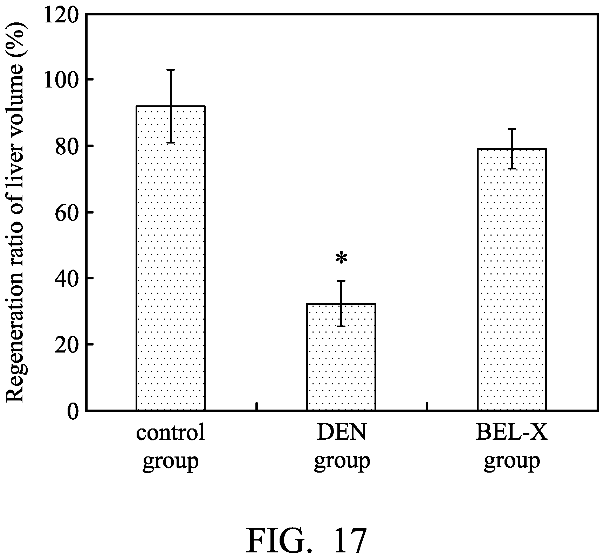

1. Referring to FIG. 17, the regeneration ratio of liver volume was interpreted. In the control group, the total liver regeneration volume of the rats with a normal liver was 92±11% of the resection volume. In DEN group (liver cirrhosis group), the total liver regeneration volume of the rats was 32±7% of the resection volume. In the BEL-X group (treatment group), the total liver regeneration volume of the rats was 79±6% of the resection volume. The degree of the liver regeneration of BEL-X group (treatment group) increased significantly to that of DEN group (liver cirrhosis group). In contrast, there is no statistical difference to that of the control group.
2. Referring to the following Table 3, after performing hepatectomy on the rats with liver cirrhosis, the eating time (27 hours) was significantly longer than the eating time (11 hours) of the control group. However, in the BEL-X group, the eating time (16 hours) after hepatectomy was significantly shorter than that of the DEN group (liver cirrhosis group). Additionally, the consumption of food (42%) of the DEN group (liver cirrhosis group) was lower than that of the control group (91%). The consumption of food (83%) of the BEL-X group as significantly higher than that of then DEN group (liver cirrhosis group), and similar to that of the control group. The results indicated that the BEL-X drug had a good effect on the liver-cirrhosis of rats after hepatectomy surgery, increasing the consumption of food and reducing the eating time.
3. Additionally, the survival rate of the rats with liver cirrhosis was 55%. However, when feeding the liver-cirrhosis rats with the BEL-X, the survival rate was the same as that of the control group, achieving 100%. The results indicated that the BEL-X drug increased the survival rate, indeed.

**Table 3**

| | Control group | DEN group | BEL-X group |
|---|---|---|---|
| Feeding amount (%) at the 3^{th} day after surgery | 91±3 | 42±5 | 83±4 |
| Eating time (hour) after surgery | 11.0±1.2 | 27.0±3.3 | 16.0±2.4 |
| Survival rate (%) | 100 | 55 | 100 |

It will be apparent to those skilled in the art that various modifications and variations can be made to the disclosed embodiments. It is intended that the specification and examples be considered as exemplary only, with the true scope of the disclosure being indicated by the following claims.

## Claims

1. A pharmaceutical composition for use as a medicament for promoting damaged liver regeneration, in a patient comprising:
a proanthocyanidin with an effective amount, wherein the monomer units of the proanthocyanidin have the following formula: 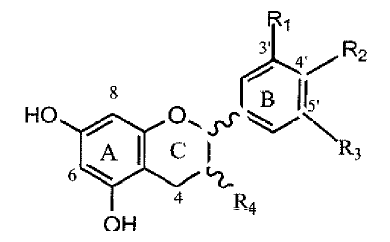 wherein, when R₁ is OCH₃, R₂ is OH and R₃ is H, when R₁ is OH, R₂ is H and R₃ is H, when R₁ is OH, R₂ is OH and R₃ is H, or when R₁ is OH, R₂ is OH and R₃ is OH, and R₄ is 3-(α)-OH, 3-(β)-OH, 3-(α)-O-sugar or 3-(β)-O-sugar; and
a pharmaceutically acceptable carrier or salt.

2. The pharmaceutical composition for use as claimed in claim 1, wherein the monomer units of the proanthocyanidin are bonded to each other via C4-C8 bonding, C4-C6 bonding, or C2-O7 bonding.

3. The pharmaceutical composition for use as claimed in claim 1, wherein the proanthocyanidin has a degree of polymerization ranging from 2 to 30.

4. The pharmaceutical composition for use as claimed in claim 1, wherein the monomer units of the proanthocyanidin comprise R or S optical isomers at C2, C3 or C4.

5. The pharmaceutical composition for use as claimed in claim 1, wherein the monomer units of the proanthocyanidin comprise flavonoid compounds.

6. The pharmaceutical composition for use as claimed in claim 5, wherein the flavonoid compounds comprise catechin, epicatechin, epiafzelechin, gallocatechin, galloepicatechin, epigallocatechin, flavonols, flavandiols, leucocyanidins or procynidins.

7. The pharmaceutical composition for use as claimed in claim 1, wherein the monomer units of the proanthocyanidin comprise flavan-3-ol.

8. The pharmaceutical composition for use as claimed in claim 1, wherein the proanthocyanidin is extracted from a plant.

9. The pharmaceutical composition for use as claimed in claim 8, wherein the plant comprises an Ericaceae, Rosaceae, Pinaceae, Vitaceae or Urticaceae plant.

10. The pharmaceutical composition for use as claimed in claim 9, wherein the Urticaceae plant comprises Boehmeria nivea L. Gaud.

## Patentansprüche

1. Pharmazeutische Zusammensetzung zur Verwendung als ein Medikament zum Fördern der Regeneration eines Leberschadens bei einem Patienten, umfassend:
ein Proanthocyanidin mit einer wirksamen Menge, wobei die Monomereinheiten des Proanthocyanidins die folgende Formel aufweisen: 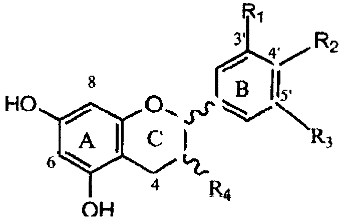 wobei, wenn R₁ OCH₃ ist, R₂ OH ist und R₃ H ist, wenn R₁ OH ist, R₂ H ist und R₃ H ist, wenn R₁ OH ist, R₂ OH ist und R₃ H ist, oder wenn R₁ OH ist, R₂ OH ist und R₃ OH ist, und R₄ 3-(α)-OH, 3-(β)-OH, 3-(α)-O-Zucker oder 3-(β)-O-Zucker ist; und einen pharmazeutisch verträglichen Träger oder Salz.

2. Pharmazeutische Zusammensetzung zur Verwendung wie in Anspruch 1 beansprucht, wobei die Monomereinheiten des Proanthocyanidins über C4-C8-Bindung, C4-C6-Bindung oder C2-O7-Bindung aneinander gebunden sind.

3. Pharmazeutische Zusammensetzung zur Verwendung wie in Anspruch 1 beansprucht, wobei das Proanthocyanidin einen Polymerisationsgrad in einem Bereich von 2 bis 30 aufweist.

4. Pharmazeutische Zusammensetzung zur Verwendung wie in Anspruch 1 beansprucht, wobei die Monomereinheiten des Proanthocyanidins optische R- oder S-Isomere an C2, C3 oder C4 umfassen.

5. Pharmazeutische Zusammensetzung zur Verwendung wie in Anspruch 1 beansprucht, wobei die Monomereinheiten des Proanthocyanidins Flavonoid-Verbindungen umfassen.

6. Pharmazeutische Zusammensetzung zur Verwendung wie in Anspruch 5 beansprucht, wobei die Flavonoid-Verbindungen Catechin, Epicatechin, Epiafzelechin, Gallocatechin, Galloepicatechin, Epigallocatechin, Flavonole, Flavandiole, Leucocyanidine oder Procynidine umfassen.

7. Pharmazeutische Zusammensetzung zur Verwendung wie in Anspruch 1 beansprucht, wobei die Monomereinheiten des Proanthocyanidins Flavan-3-ol umfassen.

8. Pharmazeutische Zusammensetzung zur Verwendung wie in Anspruch 1 beansprucht, wobei das Proanthocyanidin von einer Pflanze extrahiert ist.

9. Pharmazeutische Zusammensetzung zur Verwendung wie in Anspruch 8 beansprucht, wobei die Pflanze eine Ericaceae-, Rosaceae-, Pinaceae-, Vitaceae- oder Urticaceae-Pflanze umfasst.

10. Pharmazeutische Zusammensetzung zur Verwendung wie in Anspruch 9 beansprucht, wobei die Urticaceae-Pflanze Boehmeria nivea L. Gaud umfasst.

## Revendications

1. Composition pharmaceutique pour une utilisation comme un médicament pour favoriser une régénération d'un foie endommagé chez un patient, comprenant :
une proanthocyanidine avec une quantité efficace, dans laquelle les unités monomères de la proanthocyanidine présentent la formule suivante : 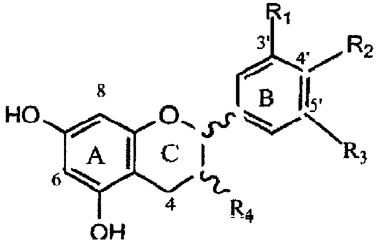 dans laquelle, lorsque R₁ est OCH₃, R₂ est OH et R₃ est H, lorsque R₁ est OH, R₂ est H et R₃ est H, lorsque R₁ est OH, R₂ est OH et R₃ est H, ou lorsque R₁ est OH, R₂ est OH et R₃ est OH, et R₄ est 3-(α)-OH, 3-(β)-OH, 3-(α)-O-sucre ou 3-(β)-O-sucre ; et
un excipient ou un sel pharmaceutiquement acceptable.

2. Composition pharmaceutique pour une utilisation selon la revendication 1, dans laquelle les unités monomères de la proanthocyanidine sont liées les unes aux autres via une liaison en C4-C8, une liaison en C4-C6, ou une liaison en C2-O7.

3. Composition pharmaceutique pour une utilisation selon la revendication 1, dans laquelle la proanthocyanidine présente un degré de polymérisation dans la plage de 2 à 30.

4. Composition pharmaceutique pour une utilisation selon la revendication 1, dans laquelle les unités monomères de la proanthocyanidine comprennent des isomères optiques R ou S au niveau de C2, C3 ou C4.

5. Composition pharmaceutique pour une utilisation selon la revendication 1, dans laquelle les unités monomères de la proanthocyanidine comprennent des composés flavonoïdes.

6. Composition pharmaceutique pour une utilisation selon la revendication 5, dans laquelle les composés flavonoïdes comprennent de la catéchine, de l'épicatéchine, de l'épiafzéléchine, de la gallocatéchine, de la galloépicatéchine, de l'épigallocatéchine, des flavanols, des flavandiols, des leucocyanidines ou des procynidines.

7. Composition pharmaceutique pour une utilisation selon la revendication 1, dans laquelle les unités monomères de la proanthocyanidine comprennent du flavan-3-ol.

8. Composition pharmaceutique pour une utilisation selon la revendication 1, dans laquelle la proanthocyanidine est extraite d'une plante.

9. Composition pharmaceutique pour une utilisation selon la revendication 8, dans laquelle la plante comprend une plante *Ericaceae, Rosaceae, Pinaceae,* Vitaceae ou Urticaceae.

10. Composition pharmaceutique pour une utilisation selon la revendication 9, dans laquelle la plante *Urticaceae* comprend de la *Boehmeria nivea* L. *Gaud.*
